# EUROPÄISCHE PATENTANMELDUNG

(11) **EP 0 984 262 A2**
(43) Veröffentlichungstag der Anmeldung: **08.03.2000**
(21) Anmeldenummer: 99115830.4
(22) Anmeldetag: 11.08.1999
(51) Int. Cl.: G01N 1/08, G01N 1/22, G01N 33/46

(54) **Vorrichtung zur Entnahme von Proben**

(30) Priorität: 13.08.1998 DE 19836633; 01.12.1998 DE 19855391
(71) Anmelder: Hofmann, Frieder, Dipl. Biologe, 28205 Bremen (DE)
(72) Erfinder: Hofmann, Frieder, Dipl. Biologe, 28205 Bremen (DE)
(74) Vertreter: von Ahsen, Erwin-Detlef, Dipl.-Ing.

(57) **Zusammenfassung**

Anhand der an der Rinde von Bäumen abgelagerten Schadstoffe wird ein Monitoring der Luftgüte, oft auch zum Nachweis von Schadstoffemissionen bestimmter Emittenten, vorgenommen. Hierfür ist die Entnahme von Proben der Baumrinde in einer genau definierten Schichtdicke erforderlich. Die erfindungsgemäße Vorrichtung zur Entnahme von Proben, insbesondere aus der Rinde von Bäumen oder anderen aktiven oder passiven Akkumulationsindikatoren für ein Luftgüte-Rindenmonitoring oder Immissions-Fingerprinting, ist durch einen Fräser (13) und einen rohrartigen Anschlag (21 bzw. 23) zum Einstellen der Frästiefe gekennzeichnet. Die Entnahmetiefe der Probe ist durch Einstellen der Frästiefe mittels des Anschlags (21 bzw. 23) genau definiert. Da der Innendurchmesser des Anschlags (21 bzw. 23) nur geringfügig größer ist als der Außendurchmesser des Fräsers (13) kann die Vorrichtung auch auf rauher bzw. grober Rinde sicher eingesetzt werden.

## Beschreibung

Die Erfindung betrifft eine Vorrichtung zur Entnahme von Proben, insbesondere aus der Rinde von Bäumen oder anderen aktiven oder passiven Akkumulationsindikatoren für das Luftgüte-Rindenmonitoring und Immissions-Fingerprinting.

Anhand von Proben, die aus einer obersten Schicht der Baumrinde entnommen werden, können Schadstoffablagerungen in der Baumrinde aus der Luft bestimmt werden. Gegenwärtig werden zu diesem Zweck Proben durch Ablösen mit einem Messer oder durch Abschaben mittels eines Schabers von der Baumrinde entnommen. Die so gewonnenen Rindenproben werden auf ihre Schadstoffbelastung hin analysiert. Hieraus lassen sich Rückschlüsse auf die Luftschadstoffbelastung am Standort des Baumes ziehen (Depositionsbelastung).

Die Rinde akkumuliert die Schadstoffe nach Art eines Input-Output-Verhältnisses. D.h. nach einer gewissen Zeit wird ein Equilibrium mit der Luftbelastung erreicht. Die äußerste Borkenschicht wird baumartspezifisch nach einer gewissen Standzeit abgestossen. Aus diesem Grund wird zur Charakterisierung der mittleren Standortbelastung eine Mischprobe über den Baumumfang und über mehrere Individuen genommen, die dann die mittlere Belastung über die Standzeit (z. B. bei Eiche 5-8 Jahre, bei Kiefer 2-3 Jahre) wiedergibt.

Hintergrund der Erfindung ist, daß aufgrund der durch das vorgenannte "Rinden-Monitoring" gewonnenen Schadstoffwerte die langjährige mittlere Immissionsdepostitionsbelastung von Standorten mit einer einmaligen Probenahme rückwirkend charakterisiert werden kann, da sich die Stoffe an der Baumrinde anlagern und diese mehrere Jahre den Immissionen ausgesetzt ist. Dies ist von Vorteil, da mit technischen Meßverfahren, wie der direkten Luftkonzentrationsmessung oder der Analyse von Niederschlägen aus Sammelgefäßen, langfristige Erfassungszeiten von mindestens 1 Jahr und mehr erforderlich sind, um ausreichend sichere Aussagen machen zu können.

Mit dem Rindenmonitoring können über das gleichzeitige Erfassen mehrerer, bestimmter charakteristischer Substanzen als sogenanntes 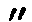 Immissions-Fingerprinting" zudem bestimmte Emittenten als Verursacher differenziert ermittelt werden.

Über das Verfahren lassen sich die Grundbelastung eines Raumes, die spezifische Zusatzbelastung von Emittenten andere Immissionseinflüsse in Relation zueinander differentialdiagnostisch genauestens bestimmen. Dies ist für zahlreiche Anwendungen im Umweltschutz relevant, die mit der Klärung von Immissionseinflüssen oder damit in Zusammenhang vermuteten Schäden befaßt sind. Treten nämlich in einem bestimmten Bereich Umweltschäden durch die Schadstoffbelastung auf, so kann für diese Schäden nach gegenwärtiger Rechtsprechung ein mitverursachender Emittent vollständig in Anspruch genommen werden, sofern sein Anteil an den zu den Umweltschäden führenden Schadstoffbelastungen als maßgeblich bewertet wird. Ein maßgeblicher Anteil wird je nach Sachlage und Rechtsprechung ab einem Anteil von 5-20 % beurteilt. Der Teilverursacher kann zwar bei anderen Mitverursachern Regressforderungen stellen; ist, wie gesagt, zunächst aber vollständig haftbar. Im Rahmen gerichtlicher Verfahren tätige Gutachter, durch die der vorgenannte Schädigungsgrad beurteilt werden soll, stehen vor dem Problem, den entsprechenden Schädigungsgrad zu ermitteln. Bisher angewendete Verfahren sind entweder unpraktikabel, weil sie sehr kostspielige und langfristige Untersuchungen erfordern oder aber sie weisen eine zu große Streuung auf, um ausreichend sichere Aussagen zu gewährleisten.

Das Verfahren bietet aufgrund der differenzierten und sicheren Bestimmung von Grund- und Zusatzbelastung breite Anwendungsmöglichkeiten für einen ermittentenbezogenen Umweltschutz, u. a. für Genehmigungsverfahren, Umweltverträglichkeitsprüfungen, Audit, Erfolgskontrollen, Immissionsüberwachung etc.

Darüber hinaus eignet sich das Verfahren auch für allgemeine gebietsbezogene Immissionsüberwachungen mit einer großen Bandbreite an räumlicher Auflösung: von lokal über regional, landes-, bundes-, europaweit bis hin zu gebietsübergreifenden globalen Vergleichen.

Das Verfahren läßt auch die Bestimmung von Depositionsraten zu und eignet sich als leistungsfähige und gleichzeitig kostengünstige Methode für das Monitoring von Schadstoffeinträgen in Waldökosysteme und allgemein der Ökosystemforschung.

Das Verfahren ist überall dort als passives Monitoring anwendbar, wo taugliche Bäume wachsen. Es läßt sich standörtlich sowohl unter Standard-Bedingungen, d. h. freie Anströmbarkeit der Bäume vergleichbar den Standortbedingungen aus den Luftmeßnetzen, als auch unter immissionsökologisch variierenden Verhältnissen zur Charakterisierung der Depositionsbelastung, z. B. innerhalb von Beständen oder an Waldrändern vergleichbar mit den Standortbedingungen aus der Waldschadensforschung, einsetzen. Da es sich standörtlich gut mit anderen Verfahren koppeln läßt kann das Verfahren eine Schlüsselrolle im sogenannten 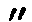 Integrierten Monitoring" übernehmen und das erforderliche Brückenglied für Vergleiche bieten.

Die Erfindung ist in dem vorstehend beschriebenen Umfeld angesiedelt und setzt bei der Probenahme an.

Der Erfindung liegt das Problem zugrunde, eine Vorrichtung zu schaffen, mit der Proben insbesondere von Baumrinden in einer genau definierten Tiefe bzw. Stärke oder Schichtdicke entnommen werden können.

Zur Lösung dieses Problems ist die erfindungsgemäße Vorrichtung durch einen Fräser und einen rohrartigen Anschlag zum Einstellen der Frästiefe gekennzeichnet, wobei der Anschlag einen Innendurchmesser aufweist, der nur geringfügig größer ist als der Außendurchmesser des Fräsers.

Der Erfindung liegt die Erkenntnis zu Grunde, daß die an verschiedenen Standorten, also an verschiedenen Bäumen zu entnehmenden Proben alle mit der selben Frästiefe entnommen werden müssen. Mit der erfindungsgemäßen Vorrichtung ist dieses auch bei sehr strukturreicher bzw. grober Borke, beispielsweise Eichen-, Eschen, Linden- oder Kieferrinde möglich, da der Innendurchmesser des Anschlags nur geringfügig größer ist als der Außendurchmesser des Fräsers und der Durchmesser des Fräsers auf die Borkenstruktur abgestimmt ist. Zwischen dem Fräser und dem Anschlag verbleibt somit nur ein sehr schmaler Spalt, so daß auch auf schmalen 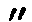 Rippen" der Rinde eine zuverlässige Frästiefeneinstellung möglich ist. Die Konzentration des sich aus der Luft ablagernden Schadstoffes in der Baumrinde nimmt nämlich mit der Eindringtiefe exponentiell ab. Zur Erlangung eines stets reproduzierbaren Ergebnisses und zur Gewährleistung der Vergleichbarkeit der Konzentrationsmessungen an unterschiedlichen Standorten muß daher die Frästiefe stets genau eingehalten werden. Nur so können Proben entnommen werden die dann einer entsprechenden Auswertung zur Identifizierung der einzelnen Immisionsquellen zu Grunde gelegt werden können.

Es sind zwar aus dem handwerklichen Bereich Oberfräsen bekannt, bei der ein Fräser durch einen rohrartigen Anschlag umschlossen wird (DE 196 37 690 A1). Der rohrartige Anschlag hat aber einen wesentlich größeren Innendurchmesser als der Außendurchmesser des Fräsers. Der Innendurchmesser des Anschlags ist sogar ein Mehrfaches des Innendurchmessers des Fräsers. Dies liegt darin begründet, daß mit der Oberfräse eine möglichst saubere, senkrecht Nut in eine ebene Platte gefräst werden muß. Hierzu ist sicherzustellen, daß die Oberfräse nicht verkantet wird, was durch einen möglichst großen Anschlagdurchmesser erreicht wird. Zum Einsatz auf strukturreichen Rinden und gekrümmten Oberflächen, wie Baumstämmen, ist die Oberfräse nicht geeignet.

Der Anschlag ist vorzugsweise durch ein Abstandrohr gegeben, dessen Stirnseite den eigentlichen Anschlag bildet. Der Fräser kann als Stirnfräser ausgebildet sein und rotiert innerhalb des Abstandsrohres. Es ergibt sich so ein den Fräser vollständig umgebender Anschlag, so daß der Anschlag in jeder Fräsposition nach Art einer Tiefenleere wirksam ist.

Weitere Merkmale der Erfindung beziehen sich auf konstruktive Ausgestaltungen. Die Erfindung wird nachfolgend anhand der Zeichnungen näher erläutert. Darin ist auch das Auswerteverfahren zur Bestimmung der Schadstoffbelastung durch einen bestimmten Emittenten näher skizziert. In den Zeichnungen zeigen:
- Fig. 1: ein Diagramm der meßbaren Schadstoffgehalte im Rindenmonitoring mit den Komponenten der Immissions-Gesamtbelastung über der Entfernung von einer bestimmten Emissionsquelle für einen bestimmten Schadstoff,
- Fig. 2: ein Diagramm der Immissions-Zusatzbelastung mit einem bestimmten Schadstoff durch verschiedene Quellen über der Entfernung von der Emissionsquelle,
- Fig. 3: ein Diagramm des Gradienten der Schadstoffgröße A für einen Fremdeinfluß über der Entfernung von der Emissionsquelle,
- Fig. 4: ein Diagramm eines Gradienten der Stoffgröße B für einen zweiten Fremdeinfluß über der Entfernung von der Emissionsquelle,
- Fig. 5: ein Diagramm der Summenparameter für weitere lokale Variationseinflüsse über der Entfernung von der Emissionsquelle,
- Fig. 6: ein Diagramm der Immissions-Zusatzbelastung des Emittenten ermittelt über ein Fingerprinting,
- Fig. 7: ein Diagramm der Immissions-Anteile des Emittenten,
- Fig. 8: ein Probenahmegerät mit den Erfindungsmerkmalen in Seitenansicht,
- Fig. 9: einen Horizontalschnitt durch das Probenahmegerät gemäß Fig. 8,
- Fig. 10: eine Vorderansicht des Probenahmegeräts gemäß Fig. 8,
- Fig. 11: unterschiedliche zu beprobende Rindentypen,
- Fig. 12: einen Querschnitt durch einen Baumstamm von Esche und Eiche,
- Fig. 13: ein Probenahmegerät mit den Erfindungsmerkmalen im Einsatz bei geschnittener Darstellung eines Baumes,
- Fig. 14: eine fotografische Darstellung des Probenahmegeräts im Einsatz,
- Fig. 15: eine weitere fotografische Darstellung des Probenahmegeräts im Einsatz,
- Fig. 16: eine fotografische Darstellung des Probenahmegeräts mit gefülltem Sammelbeutel,
- Fig. 17: eine fotografische Darstellung einer für die Probenahme geeigneten Baumgruppe,
- Fig. 18: ein Diagramm eines Vergleichs der Belastung der Luft mit Chrom, einmal anhand einer Ausbreitungsrechnung und einmal anhand der Auswertung der aus Baumrinde gewonnenen Proben.

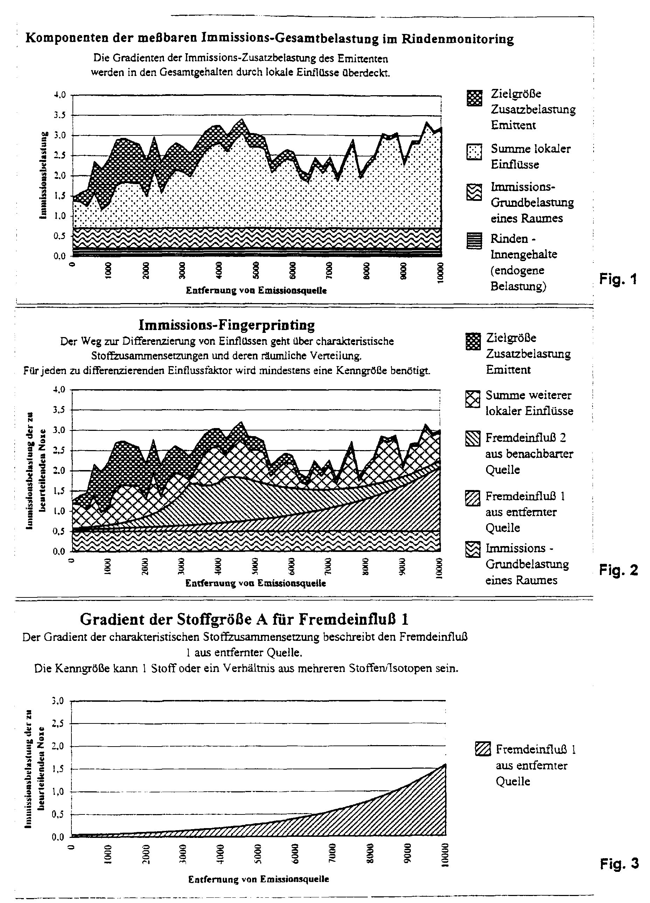

Fig. 1 zeigt ein Flächendiagramm der Immissionsbelastung für einen bestimmten Stoff. Ein solches Diagramm wird für jeden Schadstoff ermittelt. Es zeigt die Komponenten der meßbaren Gesamtbelastung für den jeweiligen Stoff aufgegliedert nach dem Rindeninnengehalt, der Immissions-Grundbelastung im Untersuchungsgebiet, die Immissions-Zusatztbelastung durch lokale Quellen und die Immissions-Zusatztbelastung durch einen bestimmten Emittenten. Aus Gründen der Anschaulichkeit wurden für diese und die folgenden Abbildungen Flächendiagramme gewählt, und somit zwischen den Standorten interpoliert.

Die Immissions-Gesamtbelastung an einem Standort entspricht den Rindengesamtgehalten (Summe aller Komponenten) abzüglich der Rindeninnengehalte (unterste Fläche mit Wellenmuster). Die Rindeninnengehalte können problemlos in einer tieferen Schicht der Baumrinde ermittelt werden, in die die Luftschadstoffe nicht eindringen können. Dieser Rindeninnengehalt entspricht dem in dem Baum vorkommenden Schadstoffgehalt. Die Immissions-Grundbelastung des Untersuchungsraumes ist als zweitunterstes jeweils quergestreift dargestellt. Die Immissions-Zusatztbelastung durch lokale Quellen ist in der Mitte diagonalschraffiert dargestellt. Im oberen Bereich befindet sich die stoffspezifische Immissions-Zusatzbelastung durch den gefragten Emittenten (gerasterte Fläche). Aufgetragen ist jeweils die Immissionsbelastung über der Entfernung von der Immissionsquelle, also von dem jeweiligen zu untersuchenden Emittenten.

Bei der Messung einer Immissionsbelastung an einem Standort mit technischen Verfahren kann stets nur die Gesamtbelastung für jeden Schadstoff ermittelt werden. Mit dem Rindenmonitoring eröffnet sich die Möglichkeit, differenziert die jeweiligen Anteile der Schadstoffquellen zu ermitteln.

Auch bei der Analyse der von der Baumrinde entnommenen Probe kann für jeden Standort zunächst erstmal nur der Gesamtgehalt gemäß Fig. 1 direkt gemessen werden. Ziel ist es letztlich, die in der erwähnten Weise dargestellte Immissions-Zusatzbelastung durch einen bestimmten Emittenten zu ermitteln, um z. B. diesen Emittenten dann gegebenenfalls für entstandene Umweltschäden in Anspruch nehmen zu können und ihn zu entlasten.

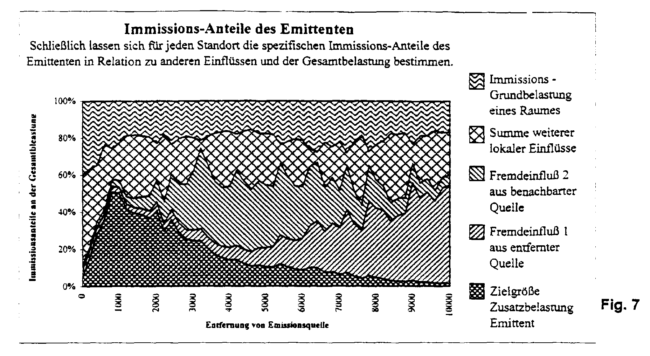

In Fig. 6 ist die Immissions-Zusatzbelastung durch den Emittenten für den vorgenannten bestimmten Schadstoff über der Entfernung aufgetragen. Es handelt sich hierbei um die bereits in Fig. 1 als Immissions-Zusatzbelastung dargestellten Werte. Man erkennt, daß sich die Immissions-Zusatzbelastung durch den bestimmten Emittenten erst in einer gewissen Entfernung, hier circa 300 Längeneinheiten, von der Immissions-Quelle bemerkbar macht und bei etwa 1000 Längeneinheiten ihr Maximum findet, um sodann mit steigender Entfernung von der Emissions-Quelle wieder abzuklingen. Diese Verteilung ist verständlich, wenn man ein Ausbreitungsdiagramm für beispielsweise eine Rauchgaswolke eines Fabrikschornsteins betrachtet. Das von ihnen emittierte Rauchgas wird vom Wind weggetragen und sinkt langsam zu Boden. Je nach Höhe des Schornsteins und der Windgeschwindigkeit und anderen meteorologischen Bedingungen gelangen die Schadstoffe erst in größerer Entfernung in Bodennähe und lagern sich dort ab. Die verschiedenen Schadstoffe sinken aufgrund ihres unterschiedlichen spezifischen Depostionsverhaltens und des unterschiedlichen Auskämmeffektes diverser Landschaftsstrukturen unterschiedlich schnell abgelagert.

Fig. 7 zeigt letztlich ein Diagramm der Immissions-Anteile des Emittenten in Relation zur Grundbelastung des Raumes und zu anderen Einflüssen in Prozent. Dieses Diagramm ist das eigentliche Ziel der Untersuchung, weil sich hieraus für jeden Standort ablesen läßt, wie hoch der spezifische Verursacheranteil des Emittenten an der Schadstoffbelastung zu bemessen ist.

Der Weg zu diesem Diagramm gemäß Fig. 7 ist in den vorhergehenden Figuren dargestellt:

Zur Ermittlung der Immissions-Zusatzbelastung durch einen bestimmten Emittenten wird die Schadstoffbelastung in der Rinde von Bäumen gemessen. Hierzu werden von den Bäumen entsprechende Proben genommen. Aus der Baumrinde werden am Umfang verteilt Proben herausgefräst. Da die Schadstoffbelastung mit der Eindringtiefe exponentiell abnimmt, ist dabei darauf zu achten, daß immer eine genau definierte Schichtstärke für alle Proben entnommen wird. Dieses wird durch ein weiter unten noch näher zu beschreibendes Probenahmegerät sichergestellt. Ferner ist bei der Probenahme darauf zu achten, daß die Schadstoffbelastung nicht durch die Probenahmetechnik, beispielsweise durch Kontamination, Verbrennen oder Verflüchtigen bestimmter Schadstoffe während der Probenahme verfälscht wird. Auch muß darauf geachtet werden, daß ein eventuell an der Rinde partiell vorkommender Bewuchs strukturreicher Moose oder Flechten oder anderer Fremdkörper nicht mitbeprobt wird. Auch dieses wird durch das weiter unter noch zu beschreibende Probenahmegerät sichergestellt bzw. ermöglicht.

Gemäß den für die jeweilige Aufgabe entsprechenden methodischen Vorgaben werden Proben an verschiedenen Standorten mit zum Teil unterschiedlicher Belastung bis hin zu ausreichend dimensionierten 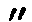 Referenzbereichen" genommen. Für eine emittentenbezogene Aufgabenstellung empfiehlt sich eine Auswahl von Standorten nach Vorgaben aus z. B. Ausbreitungsmodellen, da hierüber die z. T. sehr komplexe Gestaltung der 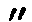 Immissionslandschaft" mit ihren Gradienten, Bergspitzen und Tälern effizient und ausreichend sicher zu erfassen ist. Aus den gewonnenen Proben werden zunächst die Rindengesamtgehalte für jeden vorhandenen Stoff analytisch gemessen. Diese entspricht dann für jeden Stoff der Gesamtfläche im Diagramm gemäß Fig. 1. Es wird nun die Immissions-Gesamtbelastung für jeden Stoff bestimmt. Dies erfolgt durch Subtraktion der endogenen Rinden-Innengehalte von den Gesamtgehalten. Diese baumeigene Belastung kann relativ einfach durch Entnahme eine Probe in einer tieferen Rindenschicht, in die Luftschadstoffe nicht mehr vordringen, gemessen werden. Dieses ist, wie gesagt, der unterste Bereich der Balken in Fig. 1 (Wellenmuster). Die nach Abzug der Rindeninnengehalte verbleibenden Gehalte stellen die durch Lufteinwirkung angelagerte Immissionsbelastung dar.

Nun wird die Immissions-Grundbelastung eines Raumes bestimmt. Dieser Wert entspricht dem niedrigsten Level der gemessenen Werte über alle Standorte in einem Untersuchungsraum. Die Größe ist raumspezifisch zu werten und stellt nach Berücksichtigung bestimmter Depositionsverhältnisse einen Basislevel dar (quergestreifte Fläche).

Als nächstes werden nun die Immissions-Zusatzbelastungen aufgrund lokal wirkender Quellen und durch den jeweils zu untersuchenden Emittenten ermittelt. Diese Zusatzbelastung setzen sich, um ein anschauliches Beispiel in Fig. 2 zu geben, aus einem ersten Fremdeinfluß aus einer entfernten Quelle (diagonalgestreift nach rechts oben), einem zweiten Fremdeinfluß aus einer benachbarten Quelle (diagonalgestreift nach links oben) und der Summe weiterer lokaler Einflüsse (kreuzgestreift, beispielsweise Heizungsanlagen in Privathäusern, verschiedener landwirtschaftlicher Bewirtschaftung, Bodenbedingungen etc.) zusammen. Die Zusatzbelastung des gefragten Emittenten stellt dann der oberste gerasterte Flächenanteil dar. Um dieses genau aufschlüsseln zu können, werden die meteorologischen Daten und der dadurch bedingte Schadstoffeintrag der verschiedenen Emissionsquellen in den zu untersuchenden Raum berücksichtigt sowie die durch die Emittenten jeweils in die Luft emittierten Schadstoffe und ihre Mengenverhältnisse zu einander. Das Stoffmuster der Emissionen bildet die Grundlage für den Immissions-Fingerabdruck jedes Emittenten. Dieser ist bei industriellen Emittenten öffentlich zugänglich. Weiterhin wird berücksichtigt, daß unterschiedliche Schadstoffe aufgrund unterschiedlicher Stoffeigenschaften schneller oder langsamer zu Boden sinken, und daß in der Luft stoffliche Veränderungen und Interaktionen von Bedeutung stattfinden können. Der zweite Weg zur Charakterisierung bestimmter Immissionseinflüsse erfolgt über eine selektive Untersuchung von Standorten und Gebieten mit bekannter Belastungsstruktur. Die dort vorgefundenen Immissionsmuster können dann die Grundlage für die differentialdiagnostische Ermittlung der wesentlichen Einflußtypen über alle Standorte sein. Unter der Bedingung, daß keine zwei Emittenten bzw. Einflüsse eine im Raum exakt gleichartige Veränderung an Stoffmustern bewirken (hierfür müßten sie die gleiche räumliche Lage und die exakt gleichen stofflichen Muster aufweisen, was äußerst unwahrscheinlich ist), dann lassen sich durch die Veränderung der Stoffmuster in den Immissionen die jeweiligen Anteile der Schadstoffquellen für jeden Standort spezifisch ermitteln Notwendig ist hierfür eine hinreichende Anzahl an bestimmender und differenzierender Parameter in der Untersuchung. Auf diese Art und Weise lassen sich die wesentlichen Immissionseinflüsse wie industrielle Emittenten, Hausbrand, Heizkraftwerke, Verkehrsbelastung, Landwirtschaft usw. sicher bestimmen.

In Fig. 3 ist die Immissionsbelastung für einen bestimmten Stoff aufgrund einer weit entfernt liegenden Quelle dargestellt. Diese Belastung nimmt verständlicher Weise mit der Entfernung zu dem bestimmten, gerade zu untersuchenden Emittenten stark zu.

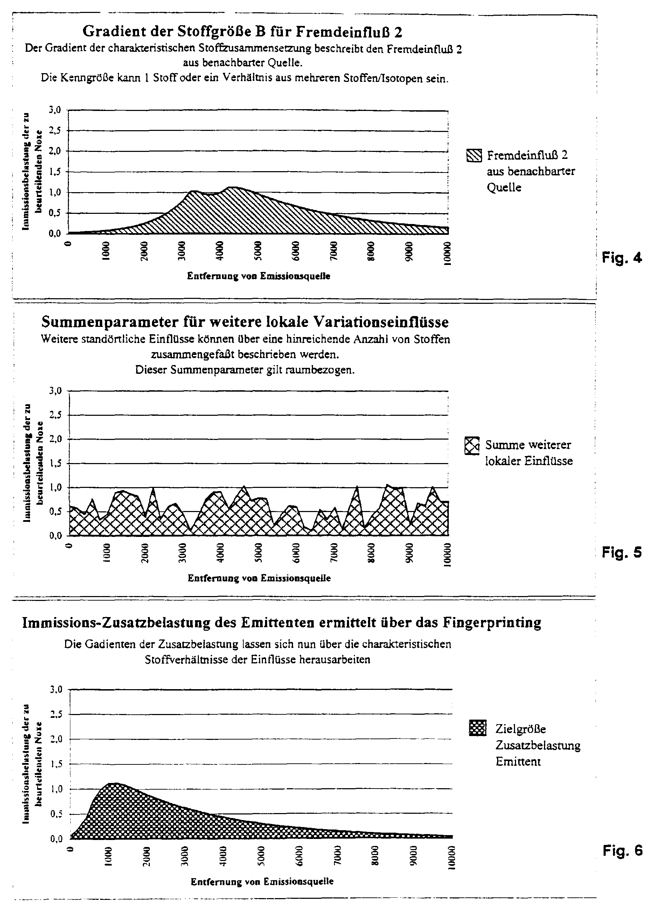

Fig. 4 zeigt beispielhaft die Zusatzbelastung durch eine in der Nähe zum zu untersuchenden Emittenten liegenden weiteren Quelle.

Fig. 5 schließlich zeigt die summarische Zusatzbelastung der weiteren, nicht näher aufgeschlüsselten lokalen Quellen über der Entfernung von dem zu untersuchenden Emittenten. Hierunter fallen auch standörtliche Einflüsse wie beispielsweise die lokale Intensität des Straßenverkehrs, die Art der landwirtschaftlichen Bewirtschaftung und auch Bodenverwehungen.

Die in den Figuren 3 bis 5 dargestellten Werte können dann jeweils in dem Diagramm gemäß Fig. 2 abgetragen werden, so daß schließlich die Zusatzbelastung durch den zu untersuchenden Emittenten übrigbleibt und gemäß Fig. 6 isoliert dargestellt werden kann.

Als letzter Schritt wird dann noch aus dem Diagramm gemäß Fig. 2 mit den absoluten Werten für die einzelnen Schadstoffbelastungen das Diagramm gemäß Fig. 7 mit den relativen Werten berechnet.

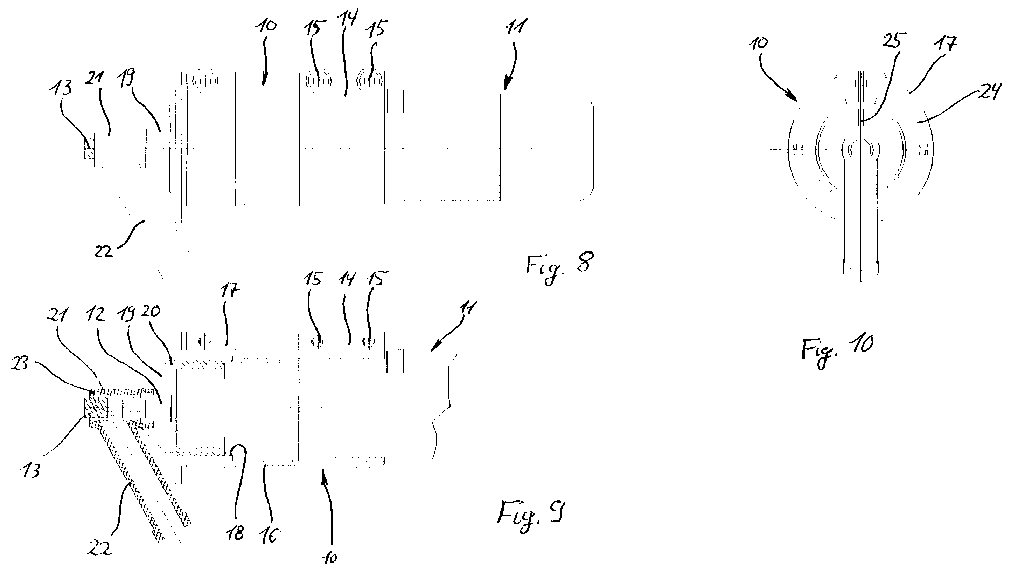

Wie bereits erwähnt, sind an die Probenahme aus der Baumrinde strenge Anforderungen zu stellen. Zum einen muß eine genau definierte Schichtstärke von der Baumrinde entnommen werden. Des weiteren hat dieses so schonend zu geschehen, daß keine chemischen Veränderungen durch Verbrennen einzelner Schadstoffe oder Verflüchtigen derselben vorkommen können. Auch Kontamination der Rinde mit Schadstoffen bei der Probenahme sollte vermieden werden. Dieses ist durch das in den Fig. 8 bis 10 näher gezeigte Probenahmengerät weitestgehend sichergestellt. Es handelt sich dabei um ein Vorsatzgerät 10, welches an einen handelsüblichen Akkuschrauber 11 befestigt wird. In das Bohrfutter 12 des Akkuschraubers 11 ist ein Stirnfräser 13 eingesetzt. Dieser dient zum Abfräsen der Proben aus der Baumrinde. Das Vorsatzgerät 10 weißt eine Klemmhülse 14 auf, die mittels zweier Schrauben 15 am Gehäuse des Akkuschraubers 11 festklemmbar ist. Über einen Rohrabschnitt 16 ist an der Klemmhülse 14 eine weitere Klemmhülse 17 einstückig angeformt. Diese besitzt ein Innengewinde 18.

Das Vorsatzgerät 10 beinhaltet weiterhin eine Einschraubhülse 19, die mit ihrem Außengewinde 20 in das Innengewinde 18 der Klemmhülse 17 einschraubbar ist. An der Anschraubhülse 19 ist ein Abstandrohr 21 fest angebracht. Das Abstandrohr 21 weißt ein Auswurfrohr 22 auf, an dem ein Auffanggefäß oder -beutel (nicht dargestellt) für die aus der Baumrinde herausgefrästen Proben befestigbar ist.

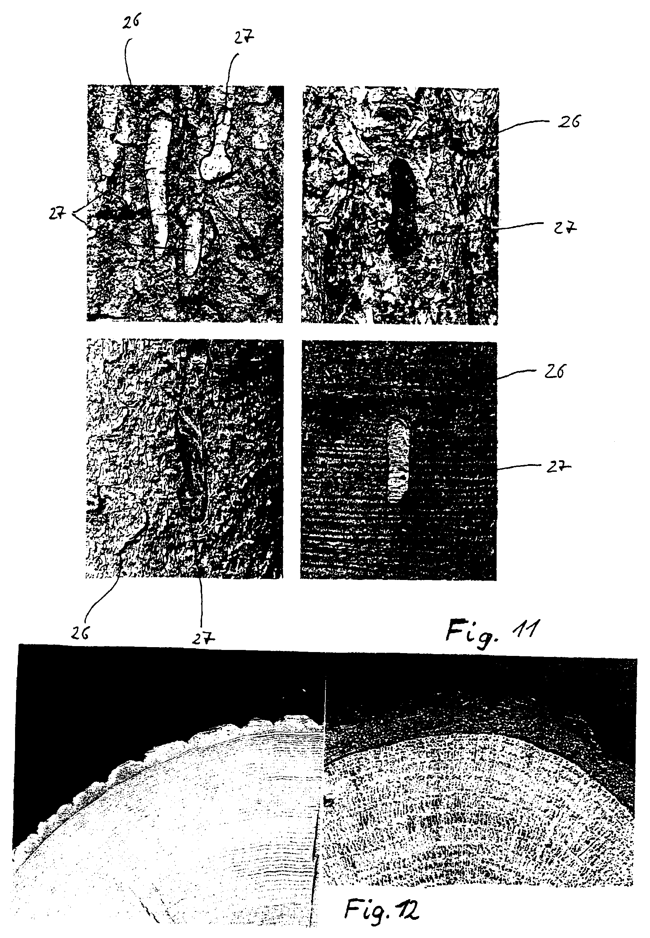

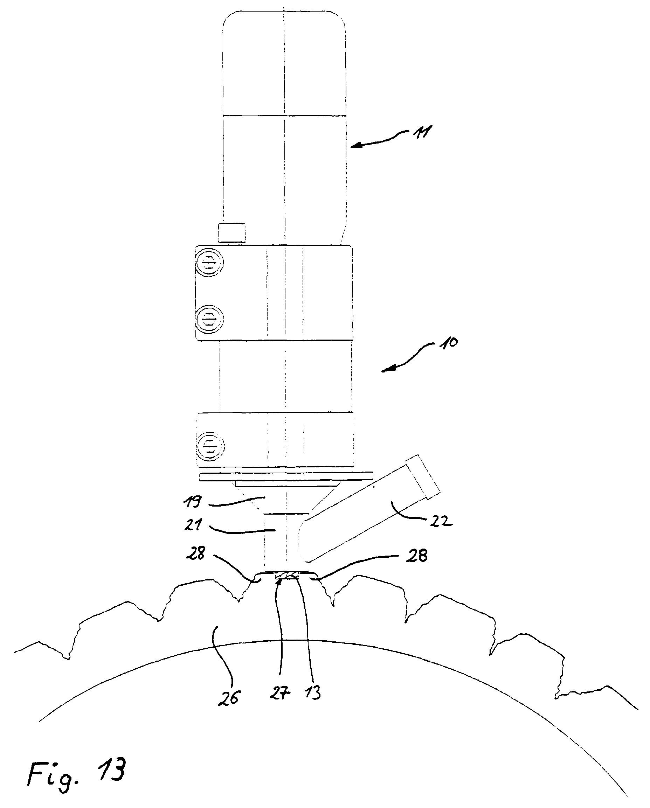

Das Abstandrohr 21 bildet mit seiner Stirnseite 23 einen Anschlag, der während des Fräsvorgangs an der Baumrinde anliegt. Das Abstandrohr 21 und damit der Anschlag 23 umschließt den Fräser 13 vollständig. Der Innendurchmesser des Abstandsrohres 21 und damit des Anschlags 23 ist nur geringfügig größer als der Außendurchmessers des Fräsers 13. Zwischen dem Fräser 13 und dem Anschlagrohr 21 verbleibt somit nur ein sehr schmaler Spalt. Hierdurch ist sichergestellt, daß die vorgegebene Frästiefe auch auf rauher Rinde 26 gut eingehalten kann. Fig. 11 zeigt einige Beispiele für unterschiedliche Rindentypen. Oben links ist die vergleichsweise rauhe Rinde von Eiche, oben rechts von Kiefer, unten links die etwas glattere Rinde von Fichte und unten rechts die noch glattere Rinde von Buche gezeigt. Auf jeder dieser Rindentypen ist eine Probenahme mit genau definierter Probentiefe, sprich Frästiefe, sicher möglich. Fig. 12 verdeutlicht die unterschiedliche Rippenstruktur von Baumrinde anhand des Beispiels von Esche (links) und Eiche (rechts). Ein Schnitt durch eine relativ rauhe Rinde 26 ist in Fig. 13 gezeigt. Es ist erkennbar, daß neben der Fräsnut 27 nur schmale Erhöhungen 28 an der Rinde 26 verbleiben, die als Auflage für den Anschlag 23 dienen können. Weiterhin wird eine Probenahme mit definierter Tiefe durch die Krümmung des Baumes erschwert. Mit dem erfindungsgemäß gestalteten Vorsatzgerät 10 ist in jeder Frässituation eine Auflage auf die Baumrinde 26 gegeben, so daß die gewünschte Frästiefe stets exakt eingehalten werden kann. Der Anschlag 23 wirkt dabei mit dem Fräser 13 nach Art einer Tiefenlehre zusammen.

Je nach Beschaffenheit der Baumrinde 26, also nach der Baumart wird ein Fräser 13 mit einem unterschiedlichen Fräserdurchmesser verwendet. Entsprechend wird auch ein Abstandsrohr 21 mit einem an den jeweiligen Durchmesser des Fräsers 13 angepaßten Durchmesser verwendet. Die Durchmesser des Fräsers 13 und des Abstandsrohres 21 werden somit auf die Beschaffenheit der Baumrinde 26, also auf die jeweilige Baumart, abgestimmt. Hierdurch ist gewährleistet, daß auch bei unterschiedlicher Beschaffenheit der Baumrinde 26 stets eine exakte Frästiefe eingehalten wird. Bei den in Fig. 12 gezeigten Beispielen für Esche einerseits und Eiche andererseits würde somit je ein unterschiedlich breiter Fräser 13 mit einem an den Fräserdurchmesser angepaßten Abstandrohr 21 verwendet werden.

Die Steigung des Außengewindes 20, der Einschraubhülse 19 und des Innengewindes 18, der Klemmhülse 17 sind im vorliegenden Fall so gewählt, daß eine Umdrehung der Einschraubhülse 19 innerhalb der Klemmhülse 17 exakt einer Axialverschiebung des Anschlags 23 um 2 mm entspricht. Um die eingestellte Frästiefe ablesen zu können, weist die Klemmhülse 17 eine Skalenscheibe 24 auf. Diese ist im vorliegenden Fall mit einer Skalierung in Schritten von 0,1 mm versehen. Unter Umständen kann auch eine feinere Skalierung gewählt werden. Mittels einer Markierung, beispielsweise einer Kerbe 25, an der Anschraubhülse 19 läßt sich so die gewünschte Frästiefe exakt einstellen. Soll eine Frästiefe von größer als 2 mm eingestellt werden, muß sich die Bedienperson merken, wie oft sie die Einschraubhülse 19 um eine volle Umdrehung gedreht hat. Der grobe Bereich läßt sich aber auch gut abschätzen bzw. mit anderen Meßinstrumenten feststellen. Die Feineinstellung auf 0,1 mm oder gar 0,01 mm läßt sich dann sehr gut mit Hilfe der Skalenscheibe 24 durchführen.

Als Antrieb dient für den vorliegenden Einsatzfall ein Akkuschrauber 11 mit einer hohen Umdrehungszahl, im vorliegenden Fall einstellbar bis zu 15.000 min⁻¹. Hierdurch wird die abgefräste Baumrinde besonders fein zermahlen und kann unmittelbar für die weitere Analyse verwendet werden. Ferner werden durch die hohe Fräsgeschwindigkeit chemische Veränderungen in der Probe, beispielsweise durch Verbrennen oder Verflüchtigen von bestimmten Schadstoffen, vermieden. Zur Vermeidung von Kontamination der Probe mit störenden Stoffen wird ein Fräser 13 aus einem Material gewählt, der keine störenden Stoffe in die Probe eintragen kann. Erforderlichenfalls werden Kreuzproben mit Fräsern 13 aus unterschiedlichem Material genommen. Ebenso ist der gesamte Materialgang bis hin zum Probenauffanggefäß aus entsprechenden Materialien gestaltet, bzw. kann der Kreuzprobe entsprechend angepaßt werden. Beispielsweise können mit Fräsern 13 aus unterschiedlichen Materialien, wie Hartmetall, Wolfram-Karbid, Titan, Magnesium und speziell vergüteten Fräsern 13 und einem Abstandsrohr 21 mit Auswurfrohr 22 aus Polyacryl sowie einem Probenahmebeutel aus Polypropylen folgende Stoffe analysiert werden:
- Elemente: Ag, Al, As, B, Ba, Be, Bi, Ca, Cd, Ce, Cr, Cs, Cu, Dy, Er, Eu, Fe, Ga, Gd, Ge, Hg, Ho, In, K, La, Li, Lu, Mg, Mn, Mo, Na, Nb, Ni, P, P, Pb, Pr, Rb, S, Sb, Se, Sm, Sn, Sr, Tb, Th, Ti, Tl, Tm, U, V, W, Yb, Zn, Zr
- Die Isotopenverhältnisse von S ( ³⁴S) und Pb (^{204, 206, 207, 208}Pb)
- Akkumulierbare organische Verbindungen wie die PAK: Acenaphthen, Acenaphthylen, Anthracen, Benzo(a)anthracen, Benzo(a)pyren, Benzo(a)fluoranthen, Benzo(g, h, i)perylen, Benzo(k)fluoranthen, Chrysen, Coronen, Dibenz(a, h)anthracen, Fluoranthen, Fluoren, Indeno (1, 2, 3-cd)pyren, Naphtalin, Phenanthren, Pyren.

Desweiteren kommen alle auf der Rinde akkumulierbaren Substanzen in Frage, u. a. aus folgenden Stoffgruppen: PCB, Dioxine, Furane, weitere nicht oder gering flüchtige halogenierte Kohlenwasserstoffe; Stickstoff N mit seinen Verbindungen Ammonium und Nitrat; stabile Isotope; radioaktive Isotope; weitere Stoffe, z. B. Platin, typische Rußbestandteile, Schwefelverbindungen.

Zudem ist eine Kühlung des Probenahmeganges durch geringe konstruktive Umgestaltung (Einleitung von Kühlgas an der Spitze) möglich, wodurch die Entnahme temperaturempfindlicher Stoffe verbessert wird.

Ferner ist das Auswurfrohr 22 nah zur Stirnfläche 23 des Abstandsrohres 21 angeordnet. Hierdurch wird der Weg der Probe in den Probenauffangbehälter kurz gehalten. Auch dies dient der Vermeidung einer Verfälschung der Probe aus analytischer Sicht, so daß ein sicherer Nachweis von Schadstoffen ohne verfälschende Einflüsse möglich ist.

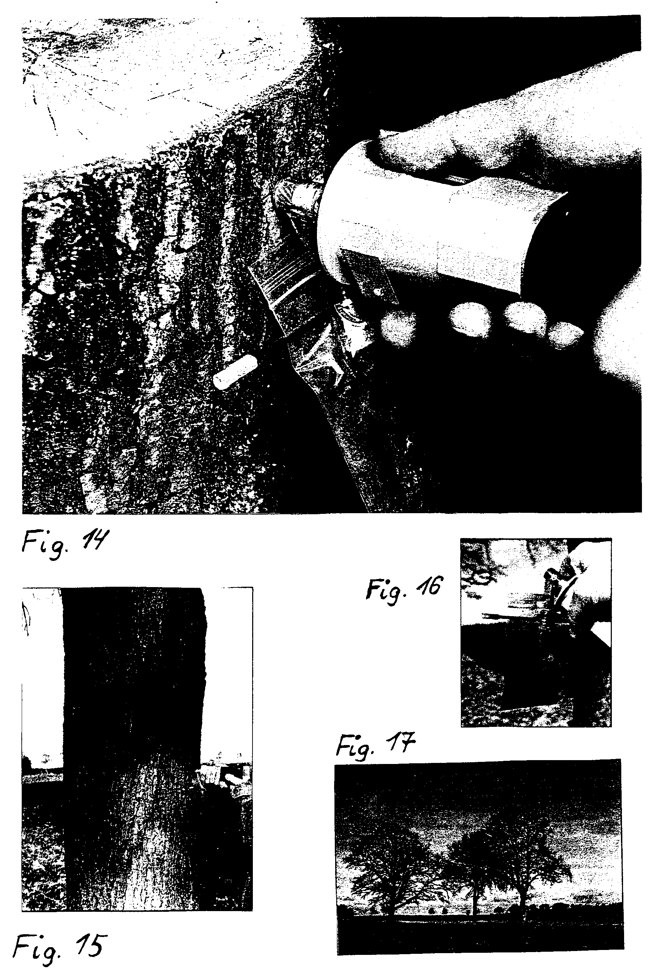

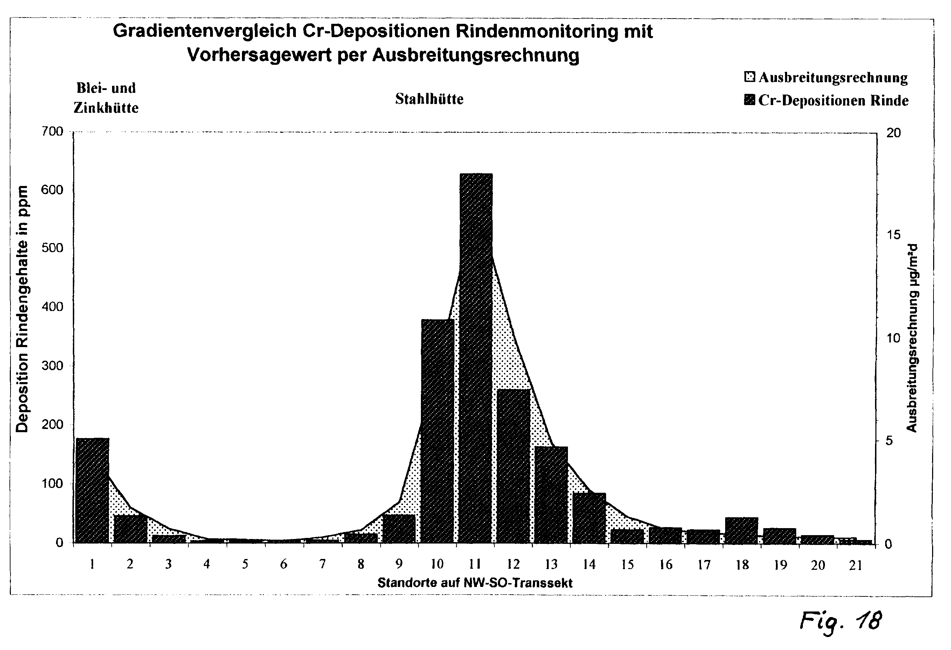

Fig. 14 zeigt das Probenahmegerät im Einsatz. Es ist erkennbar, daß mit dem Probenahmegerät entlang einer Rippe der Baumrinde gefräst wird. Das Fräsgut gelangt durch das nah an der Stirnseite 23 des Abstandsrohres angeordnete Auswurfrohr 22 direkt in den Probenahmebeutel. Das Fräsgut, sprich die Probe, ist bereits sehr fein, so daß sie unmittelbar für analytische Zwecke verwendet werden kann. Fig. 15 zeigt noch einmal das Probenahmegerät im direkten praktischen Einsatz, wobei rundherum an einem Baum, hier eine freistehende Eiche, entlang der Rippen in einer Höhe von ca. 1,5 bis 2m gefräst wird. Fig. 16 zeigt die feingemahlene Rindenprobe im Probenahmebeutel, der zugleich ohne Umfüllvorgang für den Transport ins Labor genutzt werden kann. Die Probe kann hier auch unmittelbar der Analyse zugeführt werden, ohne daß diese nochmals gemahlen werden muß. Fig. 17 zeigt frei anströmbare Bäume, hier wiederum Eichen, als Beispiel für Standorterhebung des Rindenmonitoring. Die Standortbedingungen sind durch die Wahl frei anströmbarer Bäume vergleichbar mit den Standortbedingungen der technisch genutzten Immissionsmeßnetzen.

Fig. 18 zeigt exemplarisch die Ergebnisse aus einer wissenschaftlichen Prüfung des neuen Verfahrens zum Rindenmonitoring für das Schwermetall Chrom (Cr). Deutlich sind die Gradienten für die Bleihütte (links) und die Stahlhütte (mitte) zu erkennen. Ein Vergleich von Gradienten aus dem Rindenmonitoring mit den Gradienten aus der Ausbreitungsrechnung belegt die Eignung des Rindenmonitoring für das Probenahmegerät nach der Erfindung zur Charakterisierung der Luftbelastung. Die Gradienten der Emmissionsbelastung in der Umgebung der betrachteten Emittenten können mit dem neuen Verfahren signifikant wiedergefunden werden.

### Bezugszeichenliste:

- 10: Vorsatzgerät
- 11: Akkuschrauber
- 12: Bohrfutter
- 13: Stirnfräser
- 14: Klemmhülse
- 15: Schraube
- 16: Rohrabschnitt
- 17: Klemmhülse
- 18: Innengewinde
- 19: Einschraubhülse
- 20: Außengewinde
- 21: Abstandrohr
- 22: Auswurfrohr
- 23: Stirnseite / Anschlag
- 24: Skalenscheibe
- 25: Kerbe
- 25: Rinde
- 27: Fräsnut
- 28: Erhöhungen

## Patentansprüche

1. Vorrichtung zur Entnahme von Proben, insbesondere aus der Rinde von Bäumen oder anderen aktiven oder passiven Akkumulationsindikatoren für ein Luftgüte-Rindenmonitoring oder Immissions-Fingerprinting, gekennzeichnet durch einen Fräser (13) und einen den Fräser (13) umgebenden, rohrartigen Anschlag (21 bzw. 23) zum Einstellen der Frästiefe, wobei der Anschlag (21 bzw. 23) einen Innendurchmesser aufweist, der nur geringfügig größer ist als der Außendurchmesser des Fräsers (13).

2. Vorrichtung nach Anspruch 1, dadurch gekennzeichnet, das der Anschlag als ein Anschlagrohr (21) mit einer Stirnfläche (23) als Anschlagfläche ausgebildet ist.

3. Vorrichtung nach Anspruch 1 oder 2, dadurch gekennzeichnet, das der Fräser (21) ein Stirnfräser ist.

4. Vorrichtung nach Anspruch 2 oder 3, dadurch gekennzeichnet, das der Fräser (21) innerhalb des Abstandrohres (21) rotiert und gegenüber der Stirnfläche (23) um einen einstellbaren Wert vorsteht.

5. Vorrichtung nach einem der Ansprüche 2 bis 4, dadurch gekennzeichnet, daß das Abstandsrohr (21) mittels einer Verschraubung (18, 20) gehalten ist, wobei die Frästiefe durch die Einschraubtiefe des Abstandsrohres (21) einstellbar ist.

6. Vorrichtung nach Anspruch 5, dadurch gekennzeichnet, das, die Verschraubung durch Klemmwirkung fixierbar ist.

7. Vorrichtung nach einem der Ansprüche 2 bis 6, dadurch gekennzeichnet, das an dem Abstandrohr (21) ein Auswurfrohr (22) angeordnet ist, an dem ein Probenahmebeutel zum Auffangen der herausgefrästen Proben befestigbar ist.

8. Vorrichtung nach Anspruch 7, dadurch gekennzeichnet, daß das Auswurfrohr (22) nah zur Stirnfläche (23) des Abstandsrohres (21) angeordnet ist.

9. Vorrichtung nach einem der Ansprüche 1 bis 8, dadurch gekennzeichnet, daß das Material für den Anschlag, den Fräser (13) und den Probenahmebeutel auf den in der Rinde (26) nachzuweisenden Schadstoff abgestimmt it.

10. Vorrichtung nach Anspruch 9, dadurch gekennzeichnet, daß der Fräser (13) aus einem Hartmetall, Wolfram-Karbid, Titan oder Magnesium besteht oder ein speziell vergüteter Fräser (13) ist und der Anschlag aus Polyacryl sowie der Probenahmebeutel aus Polypropylen besteht.
